(19) **Europäisches Patentamt**

**European Patent Office**

**Office européen des brevets**

(11) Veröffentlichungsnummer: **0 293 669 B1**

(12) **EUROPÄISCHE PATENTSCHRIFT**

(45) Veröffentlichungstag der Patentschrift: **23.12.92**

(21) Anmeldenummer: **88107903.2**

(22) Anmeldetag: **18.05.88**

Teilanmeldung 92103325.4 eingereicht am 18/05/88.

(51) Int. Cl.⁵: **A61M 13/00**, G05D 16/20

(54) **Gerät zum Insufflieren von Gas in eine Körperhöhle.**

(30) Priorität: **04.06.87 DE 3718717**

(43) Veröffentlichungstag der Anmeldung:
**07.12.88 Patentblatt 88/49**

(45) Bekanntmachung des Hinweises auf die Patenterteilung:
**23.12.92 Patentblatt 92/52**

(84) Benannte Vertragsstaaten:
**BE DE FR GB IT**

(56) Entgegenhaltungen:
**DE-A- 2 611 698**
**DE-A- 3 413 631**

(73) Patentinhaber: **Richard Wolf GmbH**
**Pforzheimer Strasse 32**
**W-7134 Knittlingen(DE)**

(72) Erfinder: **Baier, Manfred**
**Haydnstrasse5**
**W-7518 Bretten-Diedelsheim(DE)**
Erfinder: **Schäfer, Roland**
**Bannzaunstrasse 13**
**W-7518 Bretten-Diedelsheim(DE)**

(74) Vertreter: **Wilcken, Thomas, Dipl.-Ing. et al**
**Musterbahn 1**
**W-2400 Lübeck(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft ein Gerät zum Insufflieren von Gas in eine Körperhöhle gemäß dem Oberbegriff des Anspruches 1.

Ein spezielles Problem beim Insufflieren von Gas in eine Körperhöhle, beispielsweise während eines operativen Eingriffs, besteht darin, mit einem Gasflow einen konstanten intraabdominalen Druck innerhalb physiologisch vertretbarer Grenzen zu erzeugen und diesen Druck genau zu messen.

Bei modernen Geräten der gattungsgemäßen Art erfolgt die Zufuhr des Gases in die Körperhöhle und die Messung des statischen intraabdominalen Druckes über ein einziges Rohr. So ist beispielsweise aus der DE-C-3 000 218 ein Insufflationsgerät bekannt, bei welchem in einem Intervallbetrieb Gas durch eine Leitung insuffliert wird, danach der Gasflow abgeschaltet und daraufhin der Druck durch dieselbe Leitung gemessen wird. Diese intermittierende Methode des Gaszuflusses und der Druckmessung über eine Zuleitung führt zwangsläufig zu einer regelmäßigen Unterbrechung des Gasflusses und kann dazu führen, daß der vorzuwählende Sollwert des intraabdominalen Druckes überschritten wird, nämlich wenn eine Gasflußphase gerade vor Erreichen des Drucksollwertes einsetzt.

Ebenso ist in der Vorrichtung der EP-A-0 169 972 lediglich eine Leitung für den Gasfluß und die Druckmessung vorgesehen. In dieser Schrift wird vorgeschlagen, nach einer einmaligen, zu Beginn des gesamten Insufflationsvorganges vorgenommenen Kompensation des Systemwiderstandes, der hauptsächlich durch das in die Körperhöhle ragende Instrument, beispielsweise eine Veress-Nadel, bedingt ist, den statischen intraabdominalen Druck durch die Substraktion des Systemwiderstanddruckes vom Gesamtdruck laufend zu ermitteln und dem Arzt anzuzeigen. Dieser Vorschlag hat jedoch den Nachteil, daß eine Widerstandsänderung beispielsweise aufgrund eines Instrumentenwechsels, eines Rückgangs des Flows infolge niedriger Druckdifferenz zwischen Ein- und Ausgang oder durch Verknicken des Schlauches im weiteren Verlauf des Insufflationsvorganges unberücksichtigt bleibt.

Vor dem Hintergrund des genannten Standes der Technik ist es die Aufgabe der vorliegenden Erfindung, ein Gerät zum Insufflieren von Gas in eine Körperhöhle zu schaffen, bei welchem ständig der intraabdominale Druck gemessen wird und etwaige Veränderungen im Drucksystem während des gesamten Insufflationsvorganges bei der Messung berücksichtigt werden.

Diese Aufgabe wird bei einem Gerät der eingangs genannten Art durch die kennzeichnenden Merkmale des Anspruchs 1 gelöst.

Vorteilhafte Weiterbildungen ergeben sich aus den Unteransprüchen.

Erfindungsgemäß wird die Kompensation des Systemwiderstandes mittels einer Elektronik, die Veränderungen im Leitungssystem erkennen kann, auch zu den Zeitpunkten eingeleitet, in denen diese Veränderungen stattfinden. Dabei macht sich die Erfindung die Tatsache zunutze, daß es bei dem Betrieb eines Insufflationsgerätes verschiedene Zeitpunkte gibt, zu denen das Gerät vom Flow-Aus-Zustand in den Ein-Zustand gebracht wird, beispielsweise beim Einschalten des Gerätes, beim Konstanthalten des Solldruckes in der Körperhöhle, das durch kurze Insufflationsstöße geschieht, oder bei Veränderungen des Strömungswiderstandes infolge Instrumentenwechsels oder Flußschwankungen. Zu diesen Zeitpunkten wird der Meßschaltung ein Kompensationssignal gegengeschaltet, welches zuvor in einer Kompensationsschaltung ermittelt worden ist und welches im wesentlichen den Strömungswiderstand des das Gas in die Körperhöhle leitenden Instrumentes unmittelbar vor dem Wechsel vom Flow-Aus-Zustand in den Flow-Ein-Zustand berücksichtigt. Dieses Kompensationssignal bleibt während der gesamten Flow-Ein-Phase der Meßschaltung gegengeschaltet.

Durch die Erfindung wird demnach erreicht, daß dem Bedienungspersonal stets der tatsächliche Innendruck in der Körperhöhle auf einer Anzeige zur Kenntnis gebracht werden kann und dieser Druck für die Regelung des Gerätes auf einen Solldruck maßgebend ist.

Die Erfindung wird nachfolgend anhand der Zeichnungen näher erläutert. Es zeigen:

Figur 1      eine schematische Schaltungsanordnung des erfindungsgemäßen Gerätes, wobei im oberen Teil der Flowregelkreis und im unteren Teil der Druckregelkreis dargestellt ist,

Figur 2      eine Kompensationsschaltung zum Nullpunktabgleich eines Druckmeßwandlers, wie sie gemäß einer vorteilhaften Weiterbildung im Flowregelkreis nach Figur 1 eingesetzt wird.

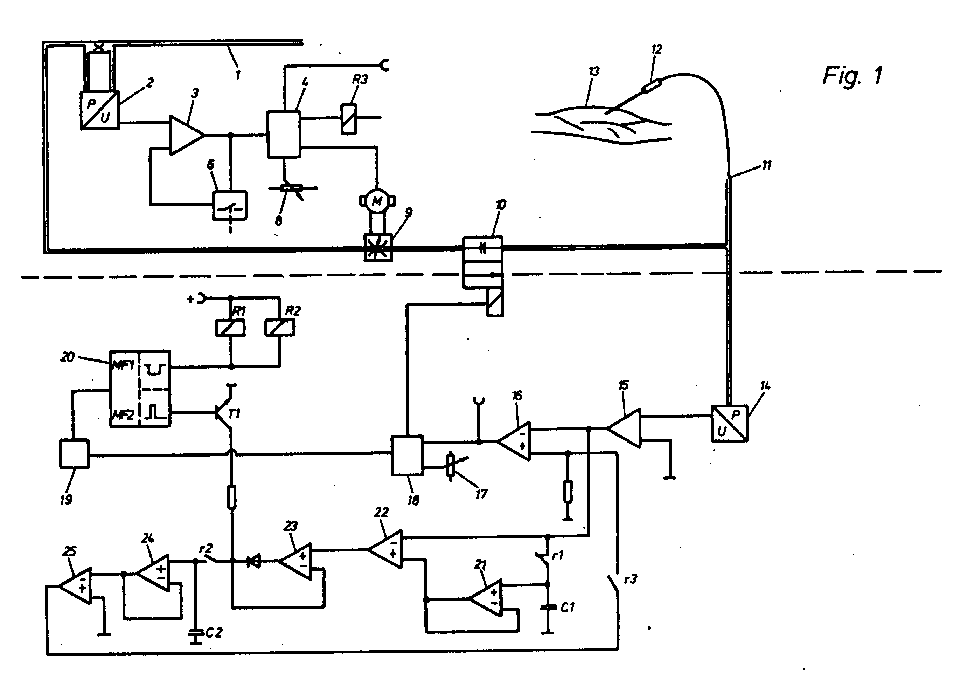

Gemäß Figur 1 wird Gas aus einem (nicht dargestellten) Druckbehälter über einen Gaseinlaß in die Gasleitung 1 des Geräts geleitet. Nach Durchgang durch die Flowregelung des Insufflators tritt das Gas aus einem Gasauslaß 11 über ein das Gas in eine Körperhöhle 13 leitendes Instrument 12, beispielsweise eine Veress-Nadel, in die Körperhöhle ein.

Die Flowregelung besteht aus einem eingangsseitig an die Gasleitung 1 angeschlossenen Differenzdruck-Meßwandler 2, dessen Ausgangssignal auf einen Eingang eines Operationsverstärkers (OP) 3 geführt ist. Dessen Ausgangssignal wird als Istwertsignal einer Regelelektronik 4 zugeführt, welche das Istwertsignal mit einem durch einen Einsteller 8 vorgewählten Sollwertsignalvergleicht.Bei einer Abweichung des Istwertes vom Sollwert des Gasflows trifft die Regelelektronik die notwendigen Maßnahmen, um den Gasflow mittels analogem Stellglied 9 an den Sollwert heranzuführen, das heißt,eine Drossel verengt oder erweitert den Durchlaß der Gasleitung 1. Über ein Ventil 10 gelangt der nunmehr konstante Gasflow an den Gasauslaß 11. Zwischen dem Ausgang des OP 3 und seinem zweiten Eingang kann eine Kompensationsschaltung 6 vorgesehen sein, die für einen Nullpunktabgleich des OP 3 bei nichtbelastetem Meßwandler 2 sorgt. Auf diese Kompensationsschaltung wird im einzelnen weiter unten eingegangen.

Der ausgangsseitig in der Gasleitung 1 herrschende Druck wird auf Seiten der Drucklegelung von einem Druckmeßwandler 14 erfaßt, der den Druck in ein entsprechendes elektrisches Signal umwandelt. Ein dem Meßwandler 14 nachgeschalteter OP 15 führt das Meßsignal an den einen Eingang eines OP 16, dessen Ausgang mit einer (nicht dargestellten) Druckanzeige sowie mit einer Auswerteelektronik 18 verbunden ist. Diese Auswerteelektronik 18 vergleicht das momentane Istwertsignal am Ausgang des OP 16 mit einem Sollwertsignal, das mittels eines Einstellers 17 auszuwählen ist. Die Auswerteelektronik 18 ist mit dem Ventil 10 verbunden, welches in Abhängigkeit von dem Resultat des Vergleiches zwischen dem Soll- und Istwertsignal in der Elektronik 18 in der Weise gesteuert wird, daß der Gasflow in der Gasleitung 1 bei Erreichen des Solldruckes in der Körperhöhle 13, das heißt also bei Gleichheit zwischen dem Istwert- und dem Sollwertsignal in 18,abgeschaltet wird.

Im Betrieb geht das Gerät zu verschiedenen Zeitpunkten vom Flow-Aus-Zustand in den Flow-Ein-Zustand über, etwa beim Einschalten, bei Konstanthalten des Solldrucks in der Körperhöhle 13, das durch kurze Insufflationsstöße geschieht, oder bei Veränderungen des Strömungswiderstandes des Drucksystems infolge eines Wechsels des Instrumentes 12 oder Flußschwankungen.

Eine an die Außwertelektronik 18 gekoppelte Steuerelektronik 19 erkennt und steuert diese Aus-Ein-Zustände. Ihr nachgeschaltet sind zwei Monoflops MF1 und MF2 in 20. Beim Übergang vom Flow-Aus-Zustand des Gerätes in den Flow-Ein-Zustand wird in der Steuerelektronik eine ansteigende Impulsflanke erzeugt, die ihrerseits die nachgeschalteten Monoflops MF1 und MF2 triggert. Dem Monoflop MF1 nachgeschaltet sind zwei Relais R1 und R2, die für die Zeitdauer des Impulses in MF1 aktiviert werden. Die Relais R1 und R2 öffnen bzw. schließen zwei Schalter r1 bzw r2 in einer Kompensationsschaltung, wie weiter unten beschrieben wird. Mit dem von MF2 erzeugten Impuls, dessen Impulsdauer wesentlich kürzer als die des Impulses im MF 1 ist, wird ein nachgeschalteter Transistor T1 durchgeschaltet. Die Funktion des T1 wird weiter unten beschrieben.

Das Ausgangssignal des OP 15 wird neben der direkten Zuleitung an den OP 16 gleichzeitig der erwähnten Kompensationsschaltung zugeführt. Zum einen wird es direkt auf einen Eingang eines OP 22 geführt,zum anderen ist es über einen durch das Relais R1 gesteuerten Schalter r1 auf einen als Impedanzwandler geschalteten OP 21 mit hohem Eingangswiderstand und geringem Ausgangswiderstand sowie auch einen zwischen dem Eingang des OP 21 und Masse geschalteten Kondensator C1 schaltbar. Der Ausgang des OP 21 ist an dem zweiten Eingang des OP 22 angeschlossen. OP 22 verstärkt die Spannungsdifferenz, deren Spitzenwert über den nachgeschalteten OP 23 mit Diode an dessem Ausgang erhalten wird. Der Ausgang des OP 23 mit Diode ist zum einen an den erwähnten Transistor T1 angeschlossen, zum anderen ist er über einen durch das Relais R2 gesteuerten Schalter r2 auf einen als Impedanzwandler geschalteten OP 24 sowie auf einen zwischen dem Eingang des OP 24 und Masse geschalteten Kondensator C2 schaltbar. Der Ausgang des OP 24 wird im nachgeschalteten OP 25 verstärkt und über einen Schalter r3, der im Flow-Aus-Zustand des Gerätes geöffnet und im Flow-Ein-Zustand geschlossen ist, an den anderen Eingang des OP 16 geführt.

Im einzelnen funktioniert die Druckmessung mit der beschriebenen Schaltungsanordnung wie folgt.

Ist der Gasflow abgeschaltet, so steht in der Gasleitung 1 ausgangsseitig der statische intraabdominale Druck, der keinerlei Verfälschungen durch einen Fließdruck erfährt. Dieser Wert wird in Form einer entsprechenden Spannung über OP 15 und OP 16 auf die Druckanzeige und gleichzeitig auf die Kompensationsschaltung gebracht, wobei der Wert über den in diesem Betriebszustand geschlossenen Schalter r1 auf den Kondensator C1 geführt und dort gespeichert wird. Der Wert wird über OP 21 an den Eingang des OP 22 geführt, dessen zweiter Eingang direkt mit der Meßspannung verbunden ist. Da eingangsseitig am OP 22 beide Werte gleich sind, erfolgt in dieser Phase keine weitere Signalverarbeitung.

Wenn nun der Gasflow wieder zugeschaltet wird, etwa wenn der Istwert des statischen intraabdominalen Druckes unter dem Sollwert liegt, werden die Schalter r1 und r2 in dieser Zuschaltphase durch die von dem Monoflop MF1 gesteuerten Relais R1 und R2 kurz geöffnet bzw. geschlossen. Gleichzeitig wird durch den vom Monoflop MF2 erzeugten Impuls über T1 und den nun geschlossenen Schalter r2 der Kondensator C2 kurzgeschlossen, damit ein in C2 bei einem vorangegangenen Zuschalten des Gasflows gespeicherter Spannungswert gelöscht wird. Mit dem Zuschalten wird der Schalter r3 geschlossen. In dieser Phase steht an dem Meßwandler 14 der statische intraabdominale Druck sowie der dynamische Druck des Gasflows, dessen Wert es zu kompensieren gilt. Der aufgrund des erhöhten Druckwertes erhöhte Spannungswert an OP 16 wird an OP 22 geführt. Da in dieser Phase der Schalter r1 geöffnet ist, wird die zuvor an C1 liegende Spannung, dem bei Flow-Aus-Zustand anlag und den Wert des statischen intraabdominalen Druckes repräsentiert, über OP 21 weiterhin an den zweiten Eingang von OP 22 geführt. Dementsprechend repräsentiert die Spannung am Ausgang vom OP 22 nur den dynamischen Teil des am Wandler anstehenden Gesamtdruckes, also den Insufflationsdruck. Der Spitzenwert der Ausgangsspannung vom OP 22 wird über OP 23 und Diode erhalten. Mit diesem Spitzenwert wird der Kondensator C 2 über den in dieser Phase geschlossenen Schalter r2 geladen. Er wird über OP 24 und OP 25 und geschlossenem Schalter r3 als Offset an den zweiten Eingang vom OP 16 geführt, an dessen Ausgang nunmehr lediglich der den statischen Anteil des Gesamtdrucks repräsentierende Spannungswert erscheint und zur Anzeige gebracht wird.

Die Dauer dieser Übergangsphase liegt im Millisekundenbereich. Danach sind die Relais R1 und R2 wieder inaktiv, das heißt, Schalter r1 wieder geschlossen und Schalter r2 wieder geöffnet. Der am Kondensator C2 gespeicherte Spannungswert wird weiterhin bis zum Ende der Insufflationsphase als Offset an OP 16 geführt. Der Wert entspricht genau der Überhöhung, die das angeschlossene Instrument 12 verursacht. Meßwerte,die nun betraglich höher als dieser Wert liegen, können nur durch einen externen Druck verursacht sein. Hier ist dies der intraabdominale Druck in der Körperhöhle 13, also der Druck, der für die Regelung des Gerätes auf den eingestellten Solldruck maßgebend ist.

Eine vorteilhafte Weiterbildung der Flowregelung besteht, wie oben erwähnt, in der Zwischenschaltung einer Kompensationsschaltung 6. Diese Kompensationsschaltung dient zum Nullabgleich des Meßwandler 2 in unbelastetem Zustand.

Wenn der Meßwandler 2 unbelastet ist, sollte sich an seinem Ausgang im Normalfall eine Spannung von 0 Volt einstellen. Dies kann aber aufgrund von Temperaturänderungen innerhalb des Gerätes infolge Wärmeentwicklung oder auch alterungsbedingt anders sein.

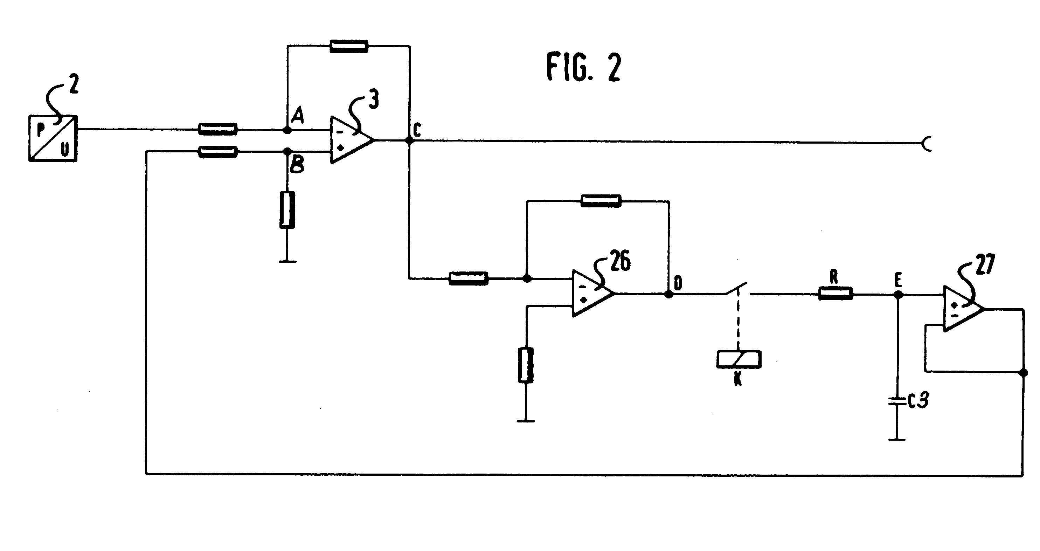

In der Schaltung nach Figur 2 wird eine von einem Druckmeßwandler 2 erzeugte Spannung an einen Eingang eines OP 3 geführt. Der Ausgang von OP 3 wird auf eine Verstärkerschaltung mit OP 26 geführt, die eine hohe Verstärkung aufweist. Das Ausgangssignal von OP 26 ist über einen Kontakt K, der immer dann geschlossen wird, wenn der Meßwandler 2 unbelastet ist, über ein Zeitglied RC auf einen elektrischen Speicher (in Figur 2:-(C3)schaltbar. Im weiteren Verlauf wird das Signal über einen OP 27, der als Impedanzwandler geschaltet ist, an den zweiten Eingang des OP 3 zurückgeführt.

Die Kompensation wird mit dem Schließen des Kontaktes aktiviert, also immer, wenn der Meßwandler 2 unbelastet ist. Dies ist dann der Fall, wenn der eingestellte Sollwert des intraabdominalen Druckes erreicht ist und der Gasflow abgeschaltet wird, da es in diesem Fall auch keinen Druckabfall mehr gibt, und gleich beim Einschalten des Gerätes.

Die Funktionsweise der Schaltung wird anhand eines Beispiels verdeutlicht:

Der Meßwandler 2 sei unbelastet und der Kontakt demnach geschlossen. Am Ausgang des Meßwandlers 2 steht eine Spannung von + 1 Volt an, am Punkt B eine Spannung von + 0,2 Volt. Der Verstärkungsfaktor von OP 3 betrage 1, von OP 26 1000.

Unter diesen Voraussetzungen ist Punkt A also um 0,8 Volt positiver als Punkt B. Demnach liegt Punkt C am Ausgang des OP 3 betragsmäßig ebenfalls auf 0,8 Volt, schaltungsbedingt aber mit negativen Vorzeichen, also minus 0,8 Volt. Da OP 26 eine hohe Verstärkung hat, würde dieser in die Sättigung fahren auf etwa 11 Volt mit positvem Vorzeichen wegen des invertierenden Verhaltens von OP 26. Mit dem am Punkt D stehenden Wert wird der KondensatorC3 über den Widerstand R geladen, wobei das RC-Glied einen langsameren Anstieg der Spannung an den punkten E und B als am Punkt D bewirkt. Dieselbe Spannung wird über OP 27 ohne Vorzeichenumkehr zum Punkt B geführt. Wenn die Spannung an den Punkten D, E und B den zu kompensierenden Wert von + 1 Volt am Punkt A erreicht hat, hört OP 26 auf, in die Sättigung von + 11 Volt zu fahren.

Da dann die Spannungswerte an den Punkten A und B gleich sind, liegt Punkt C auf Null Volt. Da OP 26 nun keine Spannung zu verstärken hat, liegt Punkt D ebenfalls auf Null Volt. An dem KondensatorC3 liegt nun eine Spannung von + 1 Volt, so daß er sich über den OP 26 wieder entladen könnte. Dies wird aber durch die geschlossene Regelschleife, wie beschrieben, ausgeglichen.

Wenn sich nun der Kontakt K öffnet, steht an dem Kondensator C3 weiterhin die Spannung von + 1 Volt an, die benötigt wird, um den Punkt C auf Null Volt auszuregeln. Tritt nun an dem Punkt A infolge eines Druckabfalls am Meßwandler 2 eine Spannung abweichend von + 1 Volt auf, wird diese als Meßspannung angesehen und weiter verarbeitet.

Der offene Regelkreis unterbindet eine weitere Kompensation. Die zuvor erhaltene Kompensationsspannung am Kondensator C3 bleibt je nach Dimensionierung von R, C3 und OP 27 für 5 bis 60 min. am Punkt B erhalten. Der beschriebene Ausregelvorgang läuft im Bereich von Millisekunden ab.

Die Schaltung kann auch dafür eingesetzt werden, um einen virtuellen Nullpunkt zu erzeugen. Beispielsweise kann ein bestimmter Druck auf den Meßwandler 2 gegeben werden und der daraus resultierende Spannungswert am Punkt A durch kurzes Schließen des Kontaktes K ausgeglichen und als Nullpunkt angesehen werden. Erst von diesem bestimmten Druck abweichende Drücke wären dann auszuwerten.

## Patentansprüche

1. Gerät zum Insufflieren von Gas in eine Körperhöhle, bei dem der dynamische Druck des zur Körperhöhle geleiteten Gasflows und der statische intraabdominale Druck in der Körperhöhle mit wenigstens einem Meßwandler (2, 14) erfaßt werden, wobei das elektrische Ausgangssignal des den Druck erfassenden Meßwandlers (14) durch Differenzbildung mit einem Kompensationssignal, das im wesentlichen den Strömungswiderstand des das Gas in die Körperhöhle leitenden Instrumentes (12) berücksichtigt, zu einem dem statischen intraabdominalen Druck entsprechenden Differenzsignal umgewandelt und wobei mit diesem Signal ein Regelkreis (18) zur Einstellung des Drucksollwertes in der Körperhöhle angesteuert wird, dadurch gekennzeichnet, daß das Kompensationssignal jeweils zu Beginn der Insufflation aus dem Ausgangssignal des betreffenden Meßwandlers (14) erzeugt und in einem ersten Speicher (C 2) gespeichert wird und daß bei Gasflow das gespeicherte Kompensationssignal dem einen Eingang und das Ausgangssignal des Meßwandlers (14) dem anderen Eingang eines ersten Operationsverstärkers (16) zur Bildung des Differenzsignales zugeführt werden, während der Speicher (C 2) eingangsseitig über einen ersten Schalter (r 2) vom Meßwandler (14) elektrisch getrennt ist.

2. Gerät nach Anspruch 1, dadurch gekennzeichnet, daß der Speicher (C 2) ein Kondensator ist, der über einen Impedanzwandler (24) mit hohem Eingangswiderstand und niedrigem Ausgangswiderstand mit dem erwähnten einen Eingang des ersten Operationsverstärkers (16) verbunden ist.

3. Gerät nach Anspruch 1 oder 2, dadurch gekennzeichnet, daß das dem intraabdominalen Drucksollwert entsprechende, vom Meßwandler (14) bei abgestelltem Gasflow erzeugte Ausgangssignal über einen geschlossenen zweiten Schalter (r 1) in einem zweiten Speicher (C 1) gespeichert und zum einen Eingang eines zweiten Operationsverstärkers (22) geleitet wird, daß mit dem Einschalten des Gasflows der zweite Schalter (r 1) kurzzeitig öffnet und das dynamische Ausgangssignal des Meßwandlers (14) auf den anderen Eingang des zweiten Operationsverstärkers (22) gegeben wird, der aus den beiden Eingangssignalen durch Differenzbildung das Kompensationssignal erzeugt, und daß das Kompensationssignal im ersten Speicher (C 2) gespeichert wird.

4. Gerät nach Anspruch 1, 2 oder 3, dadurch gekennzeichnet, daß der erste Speicher (C 2) bei jedem Einschalten des Gasflows gelöscht und anschließend mit dem Hinblick auf den Drucksollwert aktualisierten Kompensationssignal aufgeladen wird.

## Claims

1. Apparatus for insufflating gas into a body cavity, in which the dynamic pressure of the gas flow to the body cavity and the static intraabdominal pressure in the body cavity are picked up by at least one instrument transformer (2, 14), wherein the electrical output signal of the pressure picked up by the instrument transformer (14) is transformed into a differential signal, corresponding to the static intraabdominal pressure, by subtraction with a compensation signal which essentially takes into consideration the flow resistance of the instrument (12) conducting the gas into the body cavity and this signal is used for driving a control circuit (18) for adjusting the desired pressure in the body cavity, characterised in that the compensation signal is produced from the output signal of the particular instrument transformer (14) in each case at the start of insufflation and stored in a first memory (C2) and that for producing a differential signal when gas flows, the stored compensation signal is supplied to one input of a first operational amplifier (16) and the output signal of the instrument transformer (14) is supplied to the other input of a first operational amplifier (16), whilst the memory (C2) on the input side is electrically isolated from the instrument transformer (14) via a first switch (r2).

2. Apparatus according to claim 1, characterised in that the memory (C2) is a capacitor which, via an impedance transformer (24), is connected with high input resistance and low output resistance to the said input of the first operational amplifier (16).

3. Apparatus according to claim 1 or 2, characterised in that the output signal corresponding to the desired intraabdominal pressure, produced by the instrument transformer (14) when there is no gas flow, via a closed second switch (r1) is stored in a second memory (C1) and supplied to an input of the second operational amplifier (22), that with the commencement of the gas flow the second switch (r1) briefly opens and the dynamic output signal of the instrument transformer (14) is supplied to the other input of the second operational amplifier (22) which from the two input signals through subtraction produces the compensation signal, and that the compensation signal is stored in the first memory (C2).

4. Apparatus according to claim 1, 2 or 3, characterised in that the first memory (C2) is discharged with the start of each new gas flow and is then charged up with the compensation signal actualised with a view to the desired pressure.

**Revendications**

1. Appareil pour insuffler du gaz dans une cavité corporelle, dans lequel la pression dynamique du courant de gaz amené vers la cavité corporelle et la pression statique intra-abdominale à l'intérieur de la cavité corporelle sont détectées au moyen d'au moins un convertisseur de mesure (2, 14), le signal de sortie électrique du convertisseur de mesure (14) détectant la pression étant transformé, par formation de la différence avec un signal de compensation qui tient essentiellement compte de la résistance hydraulique de l'appareil (12) amenant le gaz dans la cavité corporelle, en un signal différentiel qui correspond à la pression statique intra-abdominale et sert à commander un circuit de réglage (18) pour le réglage de la valeur de consigne de la pression à l'intérieur de la cavité corporelle, **caractérisé en ce** que le signal de compensation est respectivement généré au début de l'insufflation à partir du signal du convertisseur de mesure considéré (14) et stocké dans une première mémoire (C2), et qu'en cas de circulation de gaz, le signal de compensation mémorisé est transmis à l'une des entrées et le signal de sortie du convertisseur de mesure (14), à l'autre entrée d'un premier amplificateur opérationnel (16) pour la formation du signal différentiel, tandis que du côté entrée la mémoire (C2) est électriquement isolée du convertisseur de mesure (14) par l'intermédiaire d'un premier interrupteur (r2).

2. Appareil selon la revendication 1, caractérisé en ce que la mémoire (C2) est un condensateur qui est relié à l'entrée précitée du premier amplificateur opérationnel (16) par l'intermédiaire d'un transformateur d'adaptation d'impédance (24) avec une grande résistance d'entrée et une faible résistance de sortie.

3. Appareil selon l'une des revendications 1 ou 2, caractérisé en ce que le signal de sortie correspondant à la valeur de consigne de la pression intra-abdominale et généré par le convertisseur de mesure (14) lorsque la circulation de gaz est interrompue, est stocké par l'intermédiaire d'un second interrupteur (r1) fermé dans une seconde mémoire (C1) et transmis à l'une des entrées d'un second amplificateur opérationnel (22), que la mise en circulation du gaz provoque l'ouverture temporaire du second interrupteur (r1) et l'application du signal de sortie dynamique du convertisseur de mesure (14) à l'autre entrée du second amplificateur opérationnel (22) qui, par formation de la différence, génère à partir des deux signaux d'entrée le signal de compensation, et que le signal de compensation est stocké dans la première mémoire (C2).

4. Appareil selon l'une quelconque des revendications 1, 2 ou 3, caractérisé en ce que la première mémoire (C2) est effacée à chaque mise en circulation du gaz, puis chargée avec le signal de compensation actualisé en fonction de la valeur de consigne de la pression.

Fig. 1

FIG. 2

EP 0 293 669 B1